**Europäisches Patentamt**

(19) **European Patent Office**

**Office européen des brevets**

(11) Numéro de publication: **0 026 533**

**B1**

(12) **FASCICULE DE BREVET EUROPEEN**

(45) Date de publication du fascicule du brevet: **25.05.83**

(21) Numéro de dépôt: **80200888.8**

(22) Date de dépôt: **22.09.80**

(51) Int. Cl.³: **C 07 C 53/122,**
C 07 C 53/00,
C 07 C 51/09,
C 07 C 51/46, C 07 C 51/44

(54) **Procédé pour la récupération d'acides carboxyliques à partir de mélanges contenant des esters de glycols dérivés de ces acides.**

(30) Priorité: **01.10.79 FR 7924581**

(43) Date de publication de la demande:
**08.04.81 Bulletin 81/14**

(45) Mention de la délivrance du brevet:
**25.05.83 Bulletin 83/21**

(84) Etats contractants désignés:
**AT BE CH DE FR GB IT LI NL SE**

(56) Documents cités:
**BE - A - 457 521**
**FR - A - 663 845**
**GB - A - 1 049 901**
**US - A - 4 091 039**

(73) Titulaire: **PROPYLOX (Société Anonyme)**
**12, avenue de la Renaissance**
**B-1040 Bruxelles (BE)**

(72) Inventeur: **Hardy, Nicolas**
**Rue de la Taille 52**
**B-5790 Jemeppe-sur-Sambre (BE)**

(74) Mandataire: **Meyers, Liliane et al,**
**Solvay & Cie Département de la propriété**
**industrielle 310, rue de Ransbeek**
**B-1120 Bruxelles (BE)**

Courier Press, Leamington Spa, England.

# Procédé pour la récupération d'acides carboxyliques à partir de mélanges contenant des esters de glycols dérivés de ces acides

La présente invention concerne un procédé pour la récupération d'acides carboxyliques à partir de mélanges contenant des esters de glycols dérivés de ces acides.

Elle concerne plus particulièrement la récupération d'acides carboxyliques présents dans les résidus lourds obtenus lors de l'époxydation des oléfines ay moyens des peracides carboxyliques correspondants et contenant des esters de glycols de ces acides carboxyliques.

Lors de l'époxydation des oléfines ay moyen de composés peroxydés tels que les peracides carboxyliques, on obtient un mélange contenant l'oxyde d'oléfine, l'acide carboxylique, le solvant éventuel, les réactifs non transformés ainsi que divers sous-produits dont des esters de l'acide carboxylique. Ces esters sont vraisemblablement formés par diverses réactions secondaires faisant intervenir l'oxyde d'oléfine produit et l'acide carboxylique correspondant au peracide carboxylique mis en ouevre. Ces réactions secondaires peuvent se produire tant dans le réacteur d'époxydation que dans l'installation utilisée pour la séparation du mélange issu du réacteur d'époxydation. Ainsi, lors de l'époxydation du propylène par l'acide perpropionique, on observe la formation de quantités importantes de mono- et de di-propionates de propylèneglycol, ce qui entraîne une perte de réactifs.

Pour essayer de pallier cet inconvénient, on a proposé un procédé qui vise à obtenir essentiellement du dipropionate de propylèneglycol (brevet belge 841 201 déposé le 28 avril 1976 aux noms de BAYER AG et DEGUSSA). Ce procédé ne permet évidemment pas de réduire la perte de réactifs et présente comme inconvénient la production fatale de dipropionate de propylèneglycol.

La récupération de l'acide carboxylique et de l'alcool, à partir d'un ester, par hydrolyse dans une colonne à distiller a été proposée dans le prevet BE—A—457 521 (I. G. FARBEN-INDUSTRIE AG). On a aussi proposé, dans le brevet US—A—4 091 039 (SUNTECH, Inc), d'hydrolyser des esters d'alkylèneglycols de manière à recueillir un mélange d'eau et d'acide carboxylique et d'en extraire l'acide à l'aide d'une cétone dans un extracteur-décanteur. Cette dernière technique présente l'inconvénient de ne pas permettre une récupération quantitative de l'acide et de produire des solutions d'acides carboxyliques dans une cétone qui ne sont pas substantiellement anhydres et qui ne peuvent être utilisées sans risques d'explosion pour la fabrication de peracides.

Par ailleurs, pour sécher des mélanges d'acides carboxyliques et d'eau et obtenir un acide ou un mélange d'acides exempt de tout autre constituant, on a proposé, dans le brevet FR—A—663 845 (I. G. FARBENINDUSTRIE AG), de les soumettre à une distillation extractive en présence d'un hydrocarbure chloré, comme agent d'entraînement. Dans le brevet GB—A—1 049 901 (TEKKOSHA Co. Ltd.), ce sont certaines impuretés présentes dans l'acide à épurer qui servent d'agent d'entraînement.

L'invention a pour but de fournir un procédé pour la récupération d'acides carboxyliques à partir de mélanges contenant des esters de glycols dérivés de ces acides qui évite la perte de réactifs et la production fatale d'un sous-produit.

L'invention concerne à cet effet un procédé pour la récupération d'acides carboxyliques sous forme de solutions dans un solvant organique à partir de mélanges contenant des esters de glycols dérivés de ces acides obtenus lors de l'époxydation d'une oléfine à l'aide du peracide carboxylique correspondant selon lequel on fait réagir à l'ebullition les mélanges contenant les esters avec de l'eau de manière à hydrolyser les esters et on entraîne l'acide carboxylique ainsi formé au moyen de l'eau par distillation azéotropique caractérisé en ce qu'on soumet le mélange d'acide carboxylique et d'eau sous forme de vapeur ainsi obtenu à une distillation extractive au moyen d'un solvant organique non soluble dans l'eau et dans lequel l'eau n'est pas soluble choisi parmi les esters carboxyliques, les éthers, les nitriles, les hydrocarbures halogénés, les hydrocarbures non substitués, les hydrocarbures substitués par des groupes nitro, les esters non acides d'acide carboxylique, nitrique et phosphorique et leurs mélanges de manière à séparer un mélange d'eau et de solvant organique d'une solution d'acide carboxylique dans le solvant organique.

Le procédé selon l'invention est applicable aux mélanges contenant des mono- et/ou des di-esters d'acides carboxyliques et de glycols de types divers. Il convient bien pour le traitement de mélanges contenant des esters dérivés d'acides carboxyliques contenant de 1 à 8 atomes de carbone et de glycols contenant de 2 à 4 atomes de carbone. Le procédé selon l'invention est appliqué de préférence à des mélanges contenant des esters dérivés d'acides carboxyliques contenant 1 à 4 atomes de carbone, et de glycols contenant 3 ou 4 atomes de carbone, et pouvant être substitués par des atomes d'halogènes tels que le chlore. De très bons résultats ont été obtenus en appliquant le procédé selon l'invention à des mélanges contenant au moins un composé choisi parmi le monopropionate et le dipropionate de propylèneglycol éventuellement chlorés.

La quantité totale d'esters présents dans les mélanges à traiter selon l'invention peut varier dans une large mesure selon l'origine des mélanges et les concentrations préliminaires qu'ils ont subies éventuellement. En général, les mélanges contiennent plus de 5% de leur poids d'esters.

Les mélanges à traiter selon l'invention contiennent habituellement d'autres constituants tels que les acides carboxyliques, les glycols et leurs oligomères, et des solvants.

Les acides carboxyliques pouvant être présents dans les mélanges à traiter selon l'invention peuvent être de natures diverses. Ils peuvent être de même espèce ou d'espèces différentes. En général, il s'agit d'acides carboxyliques contenant de 1 à 8 atomes de carbone. Le plus souvent, ils sont constitués principalement des acides carboxyliques dont les esters sont dérivés. De petites quantités d'autres acides carboxyliques peuvent être présentes, en général à raison de mois de 10% du poids total d'acides carboxyliques. La quantité totale d'acides carboxyliques dans les mélanges à traiter selon l'invention peut varier dans une large mesure et dépend de l'origine de ces mélanges. Elle est en général inférieur à 90% du poids des mélanges.

Les glycols et leurs oligomères pouvant être présents dans les mélanges à traiter selon l'invention peuvent être de natures diverses. Ils contiennent en général de 2 à 4 atomes de carbone substitués ou non. En général, ils sont essentiellement de même nature que ceux dont sont dérivés les esters contenus dans les mélanges à traiter. La quantité totale de glycols et de leurs oligomères est en général inférieure à 80% du poids des mélanges.

Les solvants éventuellement présents dans les mélanges à traiter peuvent être de natures très diverses. Ces solvants sont en général choisis parmi les éthers, les hydrocarbures substitués par des groupes nitro, les hydrocarbures halogénés, les hydrocarbures non substitués, les nitriles et leurs mélanges.

Les mélanges à traiter contiennent en général moins de 20% en poids de solvant et de préférence moins de 10% en poids de solvant.

Les mélanges à traiter peuvent enfin contenir également divers autres produits en quantités ne dépassant en général pas 15% de leurs poids. La nature de ces produits dépend bien entendu de l'origine des mélanges. En général, il s'agit de résidus catalytiques ainsi que de divers sous-produits des réactions ayant conduit à la formation de ces mélanges. Ainsi lorsque l'invention est appliquée à des mélanges contenant du monopropionate et/ou du dipropionate de propylèneglycol éventuellement chlorés, elle permet de récupérer aussi l'acide organique des propionates de chloropropanol ou chloroéthanol généralement présent dans ces mélanges.

Le procédé selon l'invention s'applique au traitement de mélanges à base d'esters de glycols provenant de l'époxydation des oléfines à l'intervention de peracides carboxyliques. Dans ces procédés, on obtient, après récupération de l'oxyde d'oléfine et de l'oléfine non transformée des mélanges contenant des esters dérivés des acides carboxyliques, des glycols, des acides carboxyliques et éventuellement du solvant en proportions variables selon que l'on a éliminé préalablement tout ou partie du solvant et éventuellement de l'acide carboxylique.

Si la proportion d'esters dans les mélanges à traiter est inférieure à 5% en poids ou si la proportion de solvants dépasse 10% en poids on procède en général à une évaporation préalable du solvant et éventuellement d'une partie de l'acide carboxylique de manière à accroître la proportion d'esters jusqu'à plus de 5% et de préférence jusqu'à plus de 10% en poids, ou à diminuer la proportion de solvant jusqu'à moins de 10% en poids avant de traiter les mélanges selon l'invention. Divers évaporateurs connus en eux-mêmes peuvent être utilisés à cette fin, et notamment des bouilleurs à thermosiphon, des évaporateurs à film ou des colonnes à distiller. Cette opération peut être effectuée en plusieurs étapes combinant au mieux les différents appareils. Cette vaporisation se fait en général à des températures comprises entre 300 et 420 K et à des pressions comprises entre 0,01 et 2 bar.

L'hydrolyse des esters est réalisée dans un réacteur dont la température et la pression sont tels que le mélange réactionnel est maintenu à l'ébullition. La température est en général comprise entre 300 et 450 K et la pression entre 0,05 et 10 bar. De préférence on utilise des températures de 310 à 400 K et des pressions de 0,8 à 5 bar. L'eau nécessaire est mise en ouevre sous forme liquide ou sous forme de vapeur. Il est avantageux d'introduire l'eau dans le réacteur sous forme de vapeur. Les quantités d'eau mises en oeuvre sont choisies de préférence de façon à hydrolyser les esters de manière aussi complète que possible et à entraîner en quantités aussi élevées que possible les acides carboxyliques formés par la réaction ainsi que les acides carboxyliques éventuellement présents dans les mélanges de départ. En général l'eau est mise en ouvre en quantités telles que le rapport pondéral entre l'eau et tous les acides carboxyliques présents (soit les acides déjà présents dans les mélanges à traiter et les acides formés par la réaction) soit égal ou supérieur au rapport eau sur acide carboxylique de l'azéotrope. Ainsi, lorsque l'acide carboxylique est l'acide propanoïque on l'acide butanoïque ce rapport est d'au moins environ 5:1. Pour des raisons d'économie, la quantité deau mise en oeuvre ne dépasse en général pas 10 fois la quantité d'eau nécessaire à la distillation des azéotropes eau-acide carboxylique.

On peut utiliser pour l'hydrolyse et la distillation azéotropique de l'acide carboxylique de l'eau provenant de diverses origines. Ainsi, on peut utiliser de l'eau fraîche. Lorsque le procédé de l'invention est couplé à l'époxydation d'oléfines au moyen de peracides, on utilise de façon avantageuse les eaux résiduaires obtenues lors de la concentration des effluents aqueux de la fabrication du peracide. Ces eaux résiduaires sont constituées essentiellement d'eau et contiennent de très petites quantités de catalyseurs (en général de l'acide sulfurique) et d'acide carboxylique, ne dépassant en général pas 2% de leur poids).

La réaction d'hydrolyse est en général favorisée par la présence de catalyseurs acides qui peuvent être de types très divers. L'acide sulfurique convient bien comme catalyseur. Etant donné que les mélanges à traiter contiennent le plus souvent des acides carboxyliques libres et/ou des résidus de catalyseurs acides (notamment lorsqu'ils proviennent d'une installation d'époxydation d'oléfines par les peracides) il n'est en général pas indispensable d'ajouter des catalyseurs acides.

La réaction d'hydrolyse peut être opérée en discontinu ou, de préférence, en continu dans n'importe quel type de réacteur convenant à ce genre de réaction et réalisé dans des matériaux résistant au mélange réactionnel. Lorsqu'on opère en continu, on peut utiliser des réacteurs en cascade.

Lorsqu'on opère en continu, pour éliminer les glycols formés lors de la réaction d'hydrolyse, ainsi que leurs oligomères, on purge une partie du mélange réactionnel de l'hydrolyse. Ce mélange ne contient que des quantités très faibles d'esters et d'acides carboxyliques et contient habituellement, outre les glycols et leurs oligomères, de l'eau éventuellement des catalyseurs acides et divers sous-produits. En général la purge est incinérée.

La réaction d'hydrolyse est réalisée dans des conditions où les acides carboxyliques sont entraînés par la vapeur d'eau et distillés azéotropiquement.

La phase vapeur contenant les acides carboxyliques et l'eau est soumise, selon l'invention, à une distillation extractive au moyen d'un solvant organique non soluble dans l'eau et dans lequel l'eau n'est pas soluble. Par solvents non solubles dans l'eau, on entend désigner des solvants dont la solubilité dans l'eau est inférieure à 10% et de préférence inférieure à 5%. Par solvants dans lesquels l'eau n'est pas soluble, on entend désigner ceux dans lesquels l'eau a une solubilité inférieure à 5% et de préférence inférieur à 2%. Ces solvants doivent en outre dissoudre aisément l'acide carboxylique, la solubilité de ce dernier dans le solvant devant être d'au moins 5%.

Comme solvants convenant bien, one peut citer le benzène, le toluène, le cyclohexane, le décane, l'heptane, l'éther de pétrole, le 1,2-dichloropropane, le 1,1,2,2-tétrachloréthane, le 1,2-dichloréthane, le pentachloréthane, le trichloréthylène, le tétrachloréthylène, le nitrobenzène, le chlorobenzène et le chlorure de cyclohexyle. Des résultats particulièrement bons ont été obtenus avec le 1,2-dichloréthane, le 1,2-dichloropropane le benzène et leurs mélanges.

Lorsque le procédé selon l'invention est couplé avec un procédé d'époxydation des oléfines à l'intervention de peracides carboxyliques, on utilise de façon avantageuse le même solvant que celui utilisé pour l'époxydation.

La distillation extractive ay moyen de solvant organique peut se faire selon toute technique connue en elle-même. En général, on effectue la distillation à des températures comprises entre 300 et 450 K et sous des pressions comprises entre 0,1 et 5 bar. La nouvelle phase gazeuse qui est obtenue et qui contient de l'eau et du solvant organique, quasi pur en général, est condensée et décantée selon toute technique connue en elle-même. La fraction aqueuse ainsi obtenue est rejetée et le solvant organique est recyclé à la colonne à distiller de préférence partiellement seulement. Dans ce cas, en effet, on dispose avantageusement du surplus de solvant pour la récupération par extraction liquide-liquide de l'acide carboxylique de phases aqueuses telles que celles produites, par exemple, lors de la génération du peracide carboxylique.

Au pied de la colonne à distiller on recueille les acides carboxyliques éventuellement en mélange avec le solvant.

Lorsque les mélanges à traiter selon l'invention, sont soumis préalablement à une vaporisation de manière à en séparer au moins une partie du solvant et éventuellement une partie de l'acide carboxylique, avant d'être soumis à l'hydrolyse, on utilise de façon particulièrement avantageuse la phase vapeur riche en solvant ainsi séparée pour effectuer la distillation extractive ultérieure des acides carboxyliques de leurs mélanges avec l'eau.

Le procédé selon l'invention s'applique particulièrement bien au traitement des résidus lourds contenant des esters tels que les mono- et di-propionates de glycols obtenus dans les procédés de fabrication d'oxydes d'oléfines à l'intervention de peracides carboxyliques tels que l'acide perpropionique décrits dans les brevets belges 847 664 déposé le 27 octobre 1976 aux noms de BAYER AG et DEGUSSA et 838 068 déposé le 30 janvier 1976 au nom de INTEROX CHEMICALS LTD.

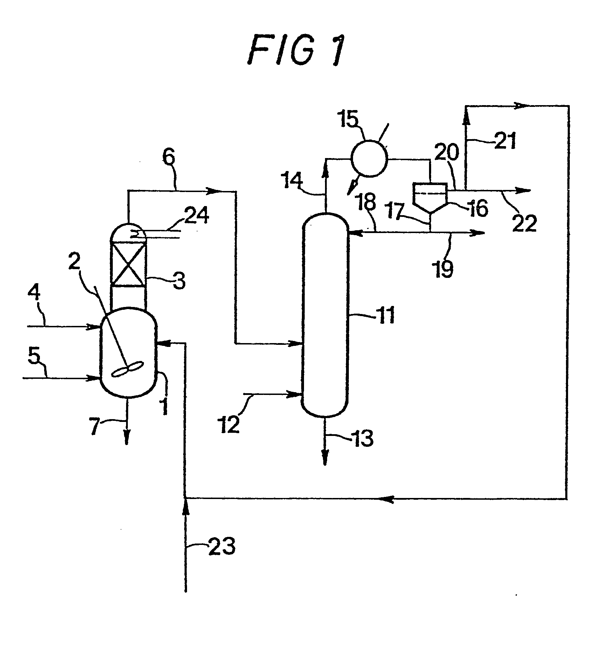

Le procédé selon l'invention peut être réalisé dans des appareils tels que ceux représentes schématiquement aux figures 1, 2 et 3 des dessins en annexe qui se rapportent à des modes de réalisation pratiques particuliers.

Selon le procédé schématisé à la figure 1, on introduit dans le réacteur 1, pourvu de moyens de chauffage (non représentés) et d'un agitateur 2 et surmonté d'une colonne à distiller 3 équipée d'un condenseur partiel 24, un mélange contenant des esters par la voie 4. De la vapeur d'eau est introduite dans le réacteur 1 par la voie 5. On soutire par la voie 6 une phase vapeur contenant l'acide carboxylique et l'eau. Par la voie 7 on soutire une purge qui contient les glycols et leurs oligomères et de l'eau. On soumet la phase vapeur contenant de l'acide carboxylique et de l'eau obtenue par la voie 6 à une distillation extractive dans la colonne à distiller 11. La colonne à distiller 11 est alimentée par la voie 12 par une phase vapeur riche en solvant organique. On recueille par la voie 13 de l'acide carboxylique généralement en mélange avec le solvant. En tête de la colonne à distiller on recueille par la voie 14 une phase vapeur contenant de l'eau et du solvant que l'on condense en 15 et que l'on envoie au décanteur 16 pour séparer la fraction aqueuse par la voie 20 de la fraction organique qui contient le solvant par la voie 17. Lorsque la denstié de la fraction aqueuse est supérieure à celle de la fraction organique, les prélèvements sont inversés. Une partie de la fraction aqueuse peut être envoyée par la voie 21 dans le réacteur d'hydrolyse; le solde est rejeté par la voie 22. Un appoint de valeur d'eau est apporté par la voie 23. La fraction organique, qui contient le solvant, est au moins partiellement recyclée à la colonne à distiller 11 où elle constitue le reflux. Le solde est recueilli par la voie 19 et peut être utilisé à la fabrication du peracide pour extraire le peracide de ses solutions aqueuses, pour extraire l'acide carboxylique de ses solutions aqueuses en encore pour servir de reflux à la colonne de distillation 3, en cas d'absence du condenseur partiel 24 (non schématisé).

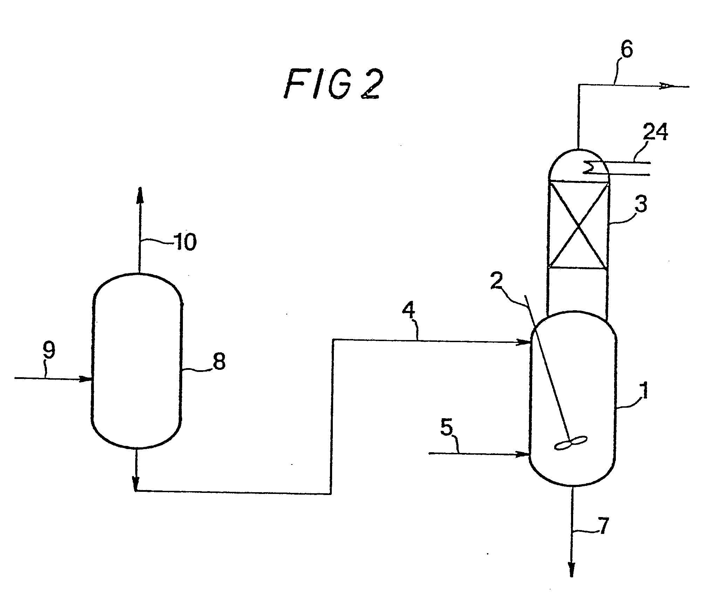

Selon le procédé schématisé à la figure 2, on introduit dans un évaporateur 8, par la voie 9 une composition contenant des esters et un solvant. On vaporise dans l'évaporateur au moins un partie du solvant que l'on recueille par la voie 10. Le mélange liquide qui contient les esters est envoyé par la voie 4 dans un appareil identique à celui schématisé à la figure 1.

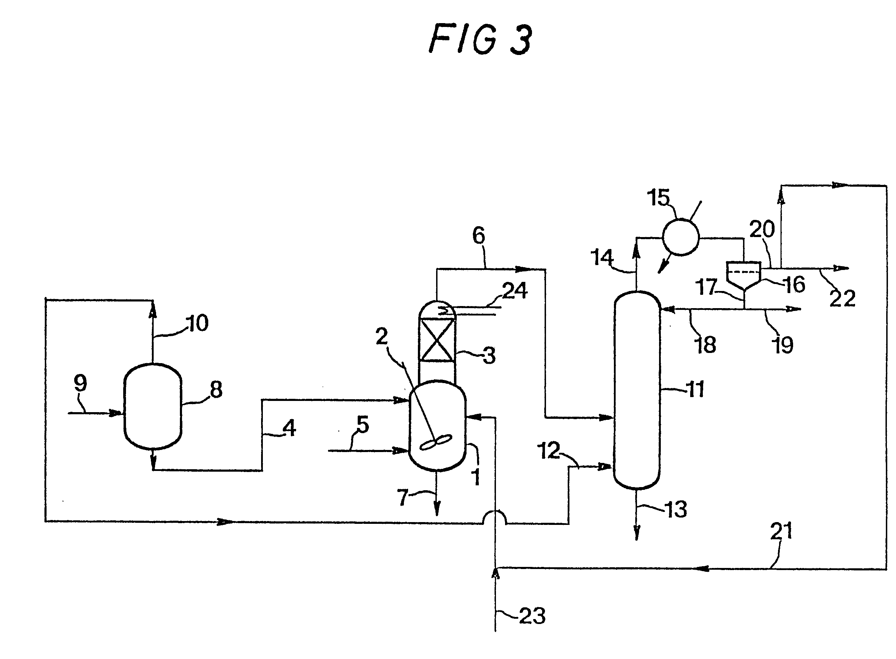

Le procédé schématisé à la figure 3 constitue une combinaison des procédés schématisés aux figures 1 et 2 selon laquelle on utilise la vapeur riche en solvant recueillie à l'évaporation 8 par la voie 10 et on l'envoie par la voie 12 à la distillation extractive 11.

Afin d'illustrer l'invention, sans pour autant en limiter la portée, on fournit ci-après un exemple pratique de réalisation.

Exemple

Cet exemple concerne le traitement d'un mélange contenant des mono- et dipropionates de propylèneglycol. Ce mélange est obtenu après séparation du propylène, de l'époxypropane et d'une partie du 1,2-dichloréthane du mélange réactionnel issu de l'époxydation du propylène par une solution d'acide perpropionique dans le dichloréthane.

Le traitement est réalisé dans l'appareil schématisé à la figure 3. Les compositions des flux de produits dans l'installation sont données au Tableau I ci-après.

On met en oeuvre environ 119 kg par heure de mélange. Le mélange est évaporé dans une section d'évaporation (8) à 2 étages. Le premier étage d'évaporation fonctionne à une température en tête de 364 K sous une pression de 0,55 bar. Le deuxième étage d'évaporation non représenté à la figure 3 fonctionne à une température en tête de 345 K sous une pression de 0,1 bar.

Le liquide recueilli au pied du second étape d'évaporation, soit 3 kg/h, est envoyé au réacteur d'hydrolyse (1) où il est traité à pression atmosphérique par 11 kg/h de vapeur d'eau. La température en tête de l'hydrolyseur (3) est de 373 K environ. On rejette en pied de l'hydrolyseur 1,5 kg de résidus par heure.

La phase gazeuse recueillie en tête de l'hydrolyseur est envoyée dans la colonne de distillation extractive (11), en même temps que le dichloréthane recueilli en tête du premier étage d'évaporation. On recueille en pied de la colonne (11), 85 kg/h d'une solution d'acide propionique dans le 1,2-dichloréthane.

La phase aqueuse évacuée par la conduite (22) contient moins de 5 g/kg d'acide propionique et la phase organique évacuée par la conduite (19) est constituée de 28 kg/h de 1,2-dichloréthane quasi pur.

La comparaison des flux dans les conduites (4) et (7) met en évidence l'efficacité de la récupération de l'acide propionique réalisée par le procédé.

TABLEAU I

| | Composition des flux en g/kg | | | | | |
|---|---|---|---|---|---|---|
| | flux 9 | flux 10 | flux 4 | flux 7 | flux 6 | flux 13 |
| eau | 0,63 | — | — | 608 | 825 | 1,5 |
| acide sulfurique | 0,09 | — | 35 | 70 | — | — |
| 1,2-dichloréthane | 733 | 769 | 20 | 20 | 10 | 653 |
| acide propionique | 232 | 220 | 171 | 83 | 150 | 315 |
| impuretés diverses | 23,95 | 7,7 | 67 | 8,7 | 12,5 | 45,27 |
| propionates de propylèneglycol | 7,8 | 2,8 | 600 | 50 | 1,1 | 3,2 |
| propylène glycol et dimères | 2,53 | 0,5 | 92 | 160,3 | 1,4 | 0,03 |

**Revendications**

1. Procédé pour la récupération d'acides carboxyliques sous forme de solutions dans un solvant organique à partir de mélanges contenant des esters de glycols dérivés de ces acides obtenus lors de l'époxydation d'une oléfine à l'aide du peracide carboxylique correspondant selon lequel on fait réagir à l'ébullition les mélanges contenant les esters avec de l'eau de manière à hydrolyser les esters et on entraîne l'acide carboxylique ainsi formé au moyen de l'eau par distillation azéotropique caractérisé en ce qu'on soumet le mélange d'acide carboxylique et d'eau sous forme de vapeur ainsi obtenu à une distillation extractive au moyen d'un solvant organique non soluble dans l'eau et dans lequel l'eau n'est pas soluble choisi parmi les esters carboxyliques, les éthers, les nitriles, les hydrocarbures halogénés, les hydrocarbures non substitués, les hydrocarbures substitués par des groupes nitro, les esters non acides d'acide carboxylique, nitrique et phosphorique et leurs mélanges de manière à séparer un mélange d'eau et de solvant organique d'une solution d'acide carboxylique dans le solvant organique.

2. Procédé selon la revendication 1 caractérisé en ce que l'on met en oeuvre des mélanges contenant des esters dérivés d'acides carboxyliques contenant 1 à 4 atomes de carbone et de glycols contenant 3 ou 4 atomes de carbone.

3. Procédé selon la revendication 1 ou 2, caractérisé en ce que l'on met en oeuvre des mélanges contenant de 5 à 80% en poids d'esters.

4. Procédé selon l'une quelconque des revendications 1 à 3 caractérise en ce que l'on met en oeuvre des mélanges contenant des acides carboxyliques.

5. Procédé selon l'une quelconque des revendications 1 à 4, caractérisé en ce que l'on met en oeuvre des mélanges contenant en outre des glycols ou leurs oligomères.

6. Procédé selon l'une quelconque des revendications 1 à 5 caractérise en ce que l'on met en oeuvre des mélanges contenant en outre un solvant.

7. Procédé selon la revendication 6 caractérisé en ce que l'on met en oeuvre des mélanges obtenus par vaporisation du solvant de manière à obtenir d'une part une fraction contenant du solvant et d'autre part des mélanges contenant les esters.

8. Procédé pour la récupération d'acides carboxyliques selon l'une quelconque des revendications 1 à 7 appliqué à un mélange contenant un solvant organique non soluble dans l'eau et dans lequel l'eau n'est pas soluble et des esters de glycols dérivés de ces acides par hydrolyse de ces esters caractérisé en ce qu'il comprend les étapes suivantes:
(a) on vaporise le solvant organique de manière à obtenir d'une part une fraction contenant du solvant sous forme de vapeur, et d'autre part des mélanges contenant les esters,
(b) on fait réagir à l'ébullition les mélanges contenant les esters avec de l'eau,
(c) on entraîne l'acide carboxylique ainsi formé au moyen de l'eau, par distillation azéotropique, et
(d) on soumet le mélange d'acide carboxylique et d'eau ainsi obtenu à une distillation extractive au moyen du solvant organique obtenu en (a) sous forme de vapeur de manière à séparer un mélange d'eau et de solvant organique d'une solution d'acide carboxylique dans le solvant organique.

9. Procédé pour la récupération d'acides carboxyliques selon l'une quelconque des revendications 1 à 7 appliqué à un mélange contenant des esters de glycols dérivés de ces acides obtenu lors de l'époxydation des oléfines à l'intervention des peracides carboxyliques, par hydrolyse de ces esters caractérisé en ce qu'il comprend les étapes suivantes:
(a) on fait réagir à l'ébullition les mélanges contenant les esters avec de l'eau résiduaire obtenue lors de la concentration des effluents aqueux de la fabrication du peracide et contenant essentiellement de l'eau, et de très petites quantités de catalyseurs et d'acide carboxylique,
(b) on entraîne l'acide carboxylique ainsi formé au moyen de l'eau, par distillation azéotropique et,
(c) on soumet le mélange d'acide carboxylique et d'eau ainsi obtenu à une distillation extractive au moyen d'un solvant organique non soluble dans l'eau et dans lequel l'eau n'est pas soluble de manière à séparer un mélange d'eau et de solvant organique d'une solution d'acide carboxylique dans le solvant organique.

10. procédé pour la récupération d'acides carboxyliques selon la revendication 8 ou 9 appliqué à un mélange contenant un solvant organique non soluble dans l'eau et dans lequel l'eau n'est pas soluble et des esters de glycols dérivés de ces acides obtenus lors de l'époxydation des oléfines par hydrolyse de ces esters caractérisé en ce qu'il comprend les étapes suivantes:
(a) on vaporise le solvant organique de manière à obtenir d'une part une fraction contenant du solvant sous forme de vapeur et d'autre part des mélanges contenant les esters,
(b) on fait réagir à l'ébullition les mélanges contenant les esters avec de l'eau résiduaire obtenue lors de la concentration des effluents aqueux de la fabrication du peracide et contenant essentiellement de l'eau, et de très petites quantités de catalyseurs et d'acide carboxylique,
(c) on entraîne l'acide carboxylique ainsi formé au moyen de l'eau, par distillation azéotropique et,
(d) on soumet le mélange d'acide carboxylique et d'eau ainsi obtenu à une distillation extractive au moyen du solvant organique obtenu en (a) sous forme de vapeur de manière à séparer un mélange d'eau et de solvant organique d'une solution d'acide carboxylique dans le solvant organique.

**Patentansprüche**

1. Verfahren zur Rückgewinnung von Carbonsäuren in Form von Lösungen in einem organischen Lösungsmittel, ausgehend von Mischungen, die Glykolesterderivate dieser Säuren enthalten, welche Mischungen bei der Epoxidation eines Olefins mit Hilfe der entsprechenden Percarbonsäure erhalten worden sind, in welchem Verfahren man bei Siedehitze die die Ester enthaltenden Mischungen mit Wasser zur Hydrolyse der Ester umsetzt und die solcherart gebildete Carbonsäure mit Hilfe des Wassers durch azeotrope Destillation abführt, dadurch gekennzeichnet, dass man das solcherart in Dampfform erhaltene Gemisch aus Carbonsäure und Wasser einer extraktiven Destillation mit Hilfe eines in Wasser nicht löslichen organischen Lösungsmittels, in welchem Wasser nicht löslich ist, unterwirft, wobei das Lösungsmittel ausgewählt ist unter den Carbonsäureestern, Ethern, Nitrilen, halogenierten Kohlenwasserstoffen, unsubstituierten Kohlenwasserstoffen, durch Nitrogruppen substituierten Kohlenwasserstoffen, nicht sauren Estern der Kohlensäure, der Salpetersäure und der Phosphorsäure und deren Gemischen, um ein Gemisch aus Wasser und dem organischen Lösungsmittel von einer Lösung der Carbonsäure in dem organischen Lösungsmittel abzutrennen.

2. Verfahren nach Anspruch 1, dadurch gekennzeichnet, dass man Mischungen einsetzt, die Esterderivate von Carbonsäuren mit 1 bis 4 Kohlenstoffatomen und von Glykolen mit 3 oder 4 Kohlenstoffatomen enthalten.

3. Verfahren nach Anspruch 1 oder 2, dadurch gekennzeichnet, dass man 5 bis 80 Gew.-% Ester enthaltende Mischungen einsetzt.

4. Verfahren nach einem der Ansprüche 1 bis 3, dadurch gekennzeichnet, dass man Carbonsäuren enthaltende Mischungen einsetzt.

5. Verfahren nach einem der Ansprüche 1 bis 4, dadurch gekennzeichnet, dass man Mischungen einsetzt, die überdies Glykole oder deren Oligomere enthalten.

6. Verfahren nach einem der Ansprüche 1 bis 5, dadurch gekennzeichnet, dass man Mischungen einsetzt, die überdies ein Lösungsmittel enthalten.

7. Verfahren nach Anspruch 6, dadurch gekennzeichnet, dass man Mischungen einsetzt, die durch derartige Verdampfung des Lösungsmittels erhalten worden sind, dass einerseits eine das Lösungsmittel enthaltende Fraktion und anderseits Mischungen gebildet werden, welche die Ester enthalten.

8. Verfahren zur Rückgewinnung von Carbonsäure nach einem der Ansprüche 1 bis 7, angewendet auf eine Mischung, die ein in Wasser nicht lösliches organisches Lösungsmittel, in welchem Wasser nicht löslich ist, sowie Glykolesterderivate dieser Säuren enthält, durch Hydrolyse dieser Ester, dadurch gekennzeichnet, dass es die folgenden Stufen umfasst:

(a) Verdampfen des organischen Lösungsmittels zur Erzielung einer das Lösungsmittel in Dampfform enthaltenden Fraktion einerseits und von die Ester enthaltenden Gemischen anderseits,

(b) Umsetzen der die Ester enthaltenden Gemische mit Wasser bei Siedehitze,

(c) Abführen der solcherart mit Hilfe des Wassers gebildeten Carbonsäure durch azeotrope Destillation, und

(d) extraktive Destillation des solcherart erhaltenen Gemisches aus Carbonsäure und Wasser mit Hilfe des in Stufe (a) in Dampfform erhaltenen organischen Lösungsmittels zur Abtrennung eines Gemisches aus Wasser und dem organischen Lösungsmittel von einer Lösung der Carbonsäure in dem organischen Lösungsmittel.

9. Verfahren zur Rückgewinnung von Carbonsäuren nach einem der Ansprüche 1 bis 7, angewendet auf eine Glykolesterderivate dieser Säuren enthaltende Mischung, die bei der Epoxidation von Olefinen unter Anwendung von Percarbonsäure erhalten wird, durch Hydrolyse dieser Ester, dadurch gekennzeichnet, dass es die folgenden Stufen umfasst:

(a) Umsetzen der die Ester enthaltenden Mischungen bei Siedehitze mit dem Restwasser, das bei der Konzentrierung der wässerigen Abströme aus der Persäureherstellung anfällt und das im wesentlichen Wasser, sehr geringe Mengen an Katalysatoren und Carbonsäure enthält,

(b) Abführen der solcherart gebildeten Carbonsäure mit Hilfe des Wassers durch azeotrope Destillation, und

(c) extraktive Destillation des solcherart erhaltenen Gemisches aus Carbonsäure und Wasser mit Hilfe eines organischen, in Wasser nicht löslichen Lösungsmittels, in welchem Wasser nicht löslich ist, zur Abtrennung eines Gemisches aus Wasser und organischem Lösungsmittel von einer Lösung der Carbonsäure in dem organischen Lösungsmittel.

10. Verfahren zur Rückgewinnung von Carbonsäuren nach Anspruch 8 oder 9, angewendet auf ein Gemisch, welches ein in Wasser nicht lösliches organisches Lösungsmittel, in welchem Wasser nicht löslich ist, und Glykolesterderivate dieser Säuren enthält und das bei der Epoxidation von Olefinen erhalten worden ist, durch Hydrolyse dieser Ester, dadurch gekennzeichnet, dass es die folgenden Stufen umfasst:

(a) Verdampfen des organischen Lösungsmittels zur Erzielung einer das Lösungsmittel in Dampfform enthaltenden Fraktion einerseits und von die Ester enthaltenden Mischungen anderseits,

(b) Umsetzen der die Ester enthaltenden Mischungen bei Siedehitze mit dem bei der Konzentrierung der wässerigen Abströme aus der Persäureherstellung erhaltenen Restwasser, das im wesentlichen Wasser und sehr geringe Mengen an Katalysatoren und Carbonsäure enthält,

(c) Abführen der solcherart gebildeten Carbonsäure mit Hilfe des Wassers durch azeotrope Destillation, und

(d) extraktive Destillation des solcherart erhaltenen Gemisches aus Carbonsäure und Wasser mit Hilfe des in Stufe (a) in Dampfform erhaltenen organischen Lösungsmittels zur Abtrennung eines Gemisches aus Wasser und organischem Lösungsmittel von einer Lösung der Carbonsäure in dem organischen Lösungsmittel.

**Claims**

1. Process for the recovery of carboxylic acids, in the form of solutions in an organic solvent, from mixtures containing glycol esters derived from these acids, and obtained during the epoxidation of an olefine with the aid of the corresponding percarboxylic acid, in which process the mixtures containing the esters are reacted at the boil with water so as to hydrolyse the esters, and the carboxylic acid thus formed is entrained by means of the water by azeotropic distillation, characterised in that the resulting mixture of carboxylic acid and water in the form of vapour is subjected to extractive distillation by means of an organic solvent which is insoluble in water and in which water is insoluble, chosen from amongst carboxylic acid esters, ethers, nitriles, halogenohydrocarbons, unsubstituted hydrocarbons, hydrocarbons substituted by nitro groups, non-acid esters of carboxylic, nitric and phosphoric acids, and mixtures thereof, so as to separate a mixture of water and organic solvent from a solution of carboxylic acid in the organic solvent.

2. Process according to Claim 1, characterised in that the mixtures used contain esters derived from carboxylic acids containing 1 to 4 carbon atoms and from glycols containing 3 or 4 carbon atoms.

3. Process according to Claim 1 or 2, characterised in that the mixtures used contain from 5 to 80% by weight of esters.

4. Process according to any one of Claims 1 to 3, characterised in that the mixtures used contain carboxylic acids.

5. Process according to any one of Claims 1 to 4, characterised in that the mixtures used also contain glycols or their oligomers.

6. Process according to any one of Claims 1 to 5, characterised in that the mixtures used also contain a solvent.

7. Process according to Claim 6, characterised in that the mixtures used are obtained by vaporising the solvent so as to give, on the one hand, a fraction containing solvent, and, on the other hand, mixtures containing the esters.

8. Process for the recovery of carboxylic acids, according to any one of Claims 1 to 7, applied to a mixture containing an organic solvent which is insoluble in water and in which water is insoluble, and glycol esters derived from these acids, by hydrolysis of these esters, characterised in that it comprises the following steps:

(a) the organic solvent is vaporised so as to give, on the one hand, a fraction containing solvent in the form of vapour, and, on the other hand, mixtures containing the esters,

(b) the mixtures containing the esters are reacted at the boil with water,

(c) the carboxylic acid thus formed is entrained by means of water by azeotropic distillation, and

(d) the mixture of carboxylic acid and water thus obtained is subjected to extractive distillation by means of the organic solvents obtained under (a) in the form of vapour, so as to separate a mixture of water and organic solvent from a solution of carboxylic acid in the organic solvent.

9. Process for the recovery of carboxylic acids, according to any one of Claims 1 to 7, applied to a mixture containing glycol esters derived from these acids, and obtained during the epoxidation of olefines with the aid of percarboxylic acids, by hydrolysis of these esters, characterised in that it comprises the following steps:

(a) the mixtures containing the esters are reacted at the boil with the waste water obtained during the concentration of aqueous effluents from the manufacture of the peracid, and essentially containing water and very small amounts of catalysts and of carboxylic acid,

(b) the carboxylic acid thus formed is entrained by means of water by azeotropic distillation, and

(c) the mixture of carboxylic acid and water thus obtained is subjected to extractive distillation by means of an organic solvent which is insoluble in water and in which water is insoluble, so as to separate a mixture of water and organic solvent from a solution of carboxylic acid in the organic solvent.

10. Process for the recovery of carboxylic acids, according to Claim 8 or 9, applied to a mixture containing an organic solvent which is insoluble in water and in which water is insoluble, and glycol esters derived from these acids, and obtained during the epoxidation of olefines, by hydrolysis of these esters, characterised in that it comprises the following steps:

(a) the organic solvent is vaporised so as to give, on the one hand, a fraction containing solvent in the form of vapour, and, on the other hand, mixtures containing the esters,

(b) the mixtures containing the esters are reacted at the boil with the waste water obtained during the concentration of the aqueous effluents from the manufacture of the peracid, and essentially containing water and very small amounts of catalysts and of carboxylic acid,

(c) the carboxylic acid thus formed is entrained by means of water by azeotropic distillation, and

(d) the mixture of carboxylic acid and water thus obtained is subjected to extractive distillation by means of the organic solvents obtained under (a) in the form of vapour, so as to separate a mixture of water and organic solvent from a solution of carboxylic acid in the organic solvent.

# FIG 1

# FIG 2

FIG 3